# EUROPÄISCHE PATENTSCHRIFT

(11) **EP 3 068 462 B1**
(45) Veröffentlichungstag und Bekanntmachung des Hinweises auf die Patenterteilung: **27.04.2022**
(21) Anmeldenummer: 14798832.3
(22) Anmeldetag: 13.11.2014
(51) Int. Cl.: A61M 5/315, A61M 5/19, A61M 5/28, A61M 5/178

(54) **SPRITZE ZUM INJIZIEREN EINER WIRKSUBSTANZ UNTER VERMEIDUNG VON TOTRAUMVERLUSTEN**
SYRINGE FOR INJECTING AN ACTIVE SUBSTANCE AVOIDING DEAD SPACE LOSSES
SERINGUE POUR INJECTER UNE SUBSTANCE ACTIVE EN ÉVITANT LES PERTES D'ESPACE MORT

(30) Priorität: 14.11.2013 DE 102013112521; 14.03.2014 DE 102014103469
(43) Veröffentlichungstag der Anmeldung: 21.09.2016
(73) Patentinhaber: Haindl, Hans, 30974 Wennigsen (DE)
(72) Erfinder: HAINDL, Hans, 30974 Wennigsen (DE)
(74) Vertreter: Vossius & Partner Patentanwälte Rechtsanwälte mbB
(86) Internationale Anmeldenummer: PCT/EP2014/074472
(87) Internationale Veröffentlichungsnummer: WO 2015/071352

(56) Entgegenhaltungen:
- WO-A1-2005/072644
- WO-A1-2010/051429
- WO-A1-2012/006555
- US-A- 4 693 706
- US-A- 6 077 252
- US-A1- 2012 265 150
- US-B1- 6 485 471
- US-B2- 7 011 650
- None

## Beschreibung

Die Erfindung bezieht sich auf eine Spritze zur Injektion einer Wirksubstanz, umfassend einen zylindrischen Spritzenkörper mit im distalen Bereich vorhandener Begrenzungswandung, die in einen Ansatz übergeht oder eine Öffnung aufweist, über den bzw. die die Wirksubstanz abgebbar ist, zwei in dem Spritzenkörper axial verschiebbare mechanisch über einen elastischen Körper verbundene Kolben und ein Betätigungselement zum gemeinsamen Verschieben der Kolben, wobei vor der Injektion der Wirksubstanz distaler Kolben und die Begrenzungswandung zueinander beabstandet sind und hierdurch gebildetes erstes Volumen mit der Wirksubstanz gefüllt ist, wobei die Kolben bei Kraftbeaufschlagung des Betätigungselementes in Richtung der Begrenzungswandung als mechanisch gekoppelte Einheit bis zum Anliegen des distalen Kolbens an der Begrenzungswandung oder einem Anschlag verstellbar sind, wobei bei mechanisch gekoppelter Einheit der Kolben einander zugewandte Innenflächen der Kolben zueinander beabstandet sind und hierdurch gebildetes zweites Volumen mit einem Nachinjiziermedium gefüllt ist und nach Anliegen des distalen Kolbens an der Begrenzungswandung bei weiterer axialer Krafteinleitung auf das Betätigungselement die mechanische Verbindung zwischen den Kolben selbsttätig derart veränderbar ist, dass proximaler Kolben in Richtung des distalen Kolbens verstellbar ist, und wobei beim Verstellen des proximalen Kolbens in Richtung des distalen Kolbens das Nachinjiziermedium den distalen Kolben durchsetzt und/oder an diesem vorbeiströmt.

In verschiedenen Bereichen der Medizin besteht der Bedarf, nach der Injektion einer Wirksubstanz eine andere Substanz nachzuinjizieren. Grund hierfür kann sein, dass die Wirksubstanz sehr teuer ist und die Menge, die sich im Totraum eines Injektionssystems, wie z. B. Butterfly-Kanüle, befindet, noch genutzt werden soll. Hierzu ist es bekannt, z. B. eine Kochsalzlösung nachzuinjizieren.

Ein anderer Grund des Nachinjizierens kann darin bestehen, dass z.B. bei Kathetern, die über eine längere Zeit liegen, nach der Gabe der Wirksubstanz das Volumen des Katheters gespült werden muss entweder weil die Wirksubstanz wie Blut in den Katheter gelangen könnte, oder weil die Wirksubstanz aggressiv ist und somit das Kathetermaterial schädigen könnte. Das Nachinjizieren erfolgt üblicherweise derart, dass eine zweite Spritze bereitgehalten wird, mittels der nach der Verabreichung der Wirksubstanz nachinjiziert wird. Dies verursacht nicht nur einen beträchtlichen Zeitaufwand, da auch die zweite Spritze aufgezogen werden muss, sondern beinhaltet auch mikrobiologische Risiken, da jeder Wechsel der Spritze mit der Gefahr des Eindringens von Keimen verbunden ist.

Der US 5 454 268 A ist eine Doppelkolbenspritze zu entnehmen, die zwei koaxial zueinander angeordnete und ineinander verschiebbare Kolben umfasst, die über eine Feder gegeneinander vorgespannt sind. Um ein Wirkmedium zu injizieren, wird zunächst der distale Kolben gegen die Federkraft in Richtung eines Kanülenansatzes verstellt. Nachdem der distale Kolben an dem Kanülenansatz anliegt, wird zunächst der über ein weiteres Federelement zurückgehaltene innere Kolben durch den distalen Kolben hindurch geschoben, um sodann in den Kanülenansatz einzudringen, um etwaiges vorhandenes Wirkmedium zu verdrängen.

Aus dem Stand der Technik sind des Weiteren Spritzen bekannt, die eine Doppelkolbenanordnung aufweisen, um z. B. ein zweites Medium zu injizieren oder Substanzen zu vermischen. So ist der EP 0 652 019 A1 eine Injektionsspritze zum Mischen und Applizieren einer Injektionssubstanz zu entnehmen. Die Spritze umfasst einen einen Stopfen aufweisenden Stempel, der ein Volumen begrenzt, in dem axial verstellbar ein Mischkolben angeordnet ist. Der Mischkolben geht von einer Kolbenstange aus, die den Stopfen durchsetzt.

Eine entsprechende Mischeinrichtung ist auch der DE 1 961 166 A1 zu entnehmen, bei der jedoch das Mischen mittels eines im Spritzenkörper fliegend angeordneten Kolbens erfolgt, der über ein Ventil verschließbar ist.

Eine Doppelkolbenanordnung mit einem fliegenden Kolben wird auch in der DE 10 209 051 863 B3 beschrieben. Der fliegende distal verlaufende Innenkolben unterteilt dabei die Spritze in zwei Kammern, so dass verschiedene Flüssigkeiten injizierbar sind.

Aus der DE 603 14 018 T2 ist eine Spritzenanordnung bekannt, in deren eine Wirksubstanz aufnehmendem Zylinder eine Kolbenstange axial verschiebbar ist, von der zwei Kolben ausgehen. Ferner sind stationäre Stopfen zum Unterteilen des Volumens des Zylinders vorgesehen.

Doppelkolbenanordnungen aufweisende Spritzen sind konstruktiv aufwendig und finden daher in der Praxis kaum Anwendung. Nachteilig ist auch, dass entsprechende Spritzen häufig bereits mit der Wirksubstanz vorbefüllt sein müssen, also ein Aufziehen vor Ort nicht möglich ist.

Der DE 43 39 528 A1 ist eine Einwegspritze zu entnehmen, die aus einem Kolben mit mittig verlaufendem Vorsprung und diesen peripher umgebender Dichtlippe besteht, die unter radialer Vorspannung dichtend an dem Spritzenzylinder anliegt. Zwischen der Dichtlippe und dem Vorsprung angesammelte Wirksubstanz kann bei axialer Krafteinleitung auf den Kolben in Richtung des Spritzenanschlusses durch Verstellen der Dichtlippe in Richtung des Vorsprungs herausgedrückt werden.

Gegenstand der DE 100 22 565 A1 ist eine Teleskopkolbenanordnung.

Eine Doppelkolbenspritze nach der WO 2005/072644 A1 weist einen proximalen und einen distalen Kolben auf, die Fluide enthaltende Volumina begrenzen, um aufeinanderfolgend diese injizieren zu können.

Bei eine Dobelkolbenanordnung nach der US 7,011,650 B2 findet sich in einem Spritzenkörper ein Art Faltenbalg, in dem eine Wirksubstanz enthalten ist, die durch Zusammendrücken des Faltenbalgs injiziert wird.

Innerhalb eines Spritzenkörpers ist nach der US 6,077,252 eine faltenbalgartige Kartusche zusammendrückbar angeordnet, um eine Kochsalzlösung injizieren zu können.

Gegenstand der US 6,485,471 B1 ist eine Spritze, innerhalb der ein faltenbalgartiges Behältnis angeordnet ist, in dem sich eine medizinische Lösung befindet.

Um Flüssigkeiten zu mischen, sieht die US 4,693,706 A eine Doppelkolbenanordnung vor.

Der vorliegenden Erfindung liegt die Aufgabe zu Grunde, eine Spritze vorzuschlagen, mit der problemlos Restmengen von Wirksubstanzen aus der Spritze injiziert werden können, ohne dass ein Vorbefüllen am Produktionsort der Wirksubstanz zwingend erforderlich ist. Dabei soll die Spritze konstruktiv einfach aufgebaut und somit kostengünstig herstellbar sein.

Die Aufgabe wird gelöst durch den Gegenstand von Anspruch 1. Weitere Ausführungsformen sind in den abhängigen Ansprüchen angegeben.

Zur Lösung der Aufgabe sieht die Erfindung vor, dass das zweite Volumen mit einem Gas als das Nachinjiziermedium gefüllt ist und dass nach dem Injizieren des Nachinjiziermediums ein Zurückgleiten des proximalen Kolbens durch Klemmung oder Reibschluss oder durch im von den Kolben und dem diese verbindenden elastischen Körper umgebenen Raum herrschenden Unterdruck unterbunden ist, wobei dann, wenn das Zurückgleiten durch den Unterdruck unterbunden ist, der distale Kolben in seiner distal verlaufenden Begrenzung zumindest eine Öffnung aufweist, die über ein Rückschlagventil verschlossen ist.

Erfindungsgemäß wird eine Doppelkolbenspritze benutzt, um gezielt ein Wirkmedium aus dem Totvolumen der Spritze bzw. einer mit dieser verbundenen Kanüle oder eines Katheters zu spülen. Dabei ist es nicht erforderlich, dass das erste Volumen, also der Bereich zwischen Ansatz und distal verlaufender Stirnfläche des distalen Kolbens, vorbefüllt wird. Vielmehr besteht die Möglichkeit, vor Ort die Wirksubstanz, also ein Medikament, aufzuziehen. Des Weiteren sollte sich als Medium zwischen den Kolben sterile Luft befinden, mittels der das Spülen erfolgt.

Aufgrund der mechanischen Kopplung zwischen den Kolben kann problemlos eine Wirksubstanz aufgezogen werden, ohne dass das zwischen den Kolben verlaufende zweite Volumen und somit die sterile Luft entweicht. Bei Benutzung wird sodann durch axiale Krafteinleitung in Richtung des Ansatzes die aus den Kolben bestehende Einheit axial verstellt, um die Wirksubstanz zu injizieren. Nachdem der distale Kolben an dem Ansatz bzw. einer in den Ansatz übergehenden Begrenzungswandung oder einem sonstigen Anschlag anliegt, wird die mechanische Verbindung zwischen den Kolben aufgehoben, um sodann den proximalen Kolben in Richtung des distalen Kolbens mit der Folge verstellen zu können, dass das in dem zweiten Volumen vorhandene Medium wie sterile Luft über den distalen Kolbenansatz und damit einer Kanüle oder einem Katheter zugeführt wird. Somit werden Kanüle bzw. Katheter oder ein sonstiges Schlauchsystem durch die Luft freigespült.

Durch die zwingende mechanische Kopplung der Kolben ist im Vergleich zu fliegenden Kolben sichergestellt, dass eine definierte Menge an Medium, also insbesondere an steriler Luft, nachinjiziert wird. Besondere Kraftaufwendungen zum Nachinjizieren sind im Vergleich zu Doppelkolbenkonstruktionen nicht erforderlich, bei denen die Kolben gegeneinander federvorgespannt sind, um ein sukzessives Bewegen zunächst der Einheit aus ersten und zweiten Kolben und sodann des proximalen Kolbens zu ermöglichen, wie dies die US 5 454 268 A vorsieht, ohne dass die Möglichkeit eines Nachinjizierens besteht.

Insbesondere sieht die Erfindung vor, dass der distale Kolben ein Hohlkolben mit distal verlaufender Bodenwandung und von dieser ausgehender einen Hohlzylinder bildender Umfangswandung mit abschnittsweise radial in Richtung der Längsachse des Kolbens sich erstreckendem Randbereich, insbesondere einem umlaufenden nach innen abgewinkelten Rand, ist, der in eine den Randbereich aufnehmende in der Außenseite des proximalen Kolbens axial verlaufende Aussparung eingreift, die beabstandet zu zumindest distalem Rand des proximalen Kolbens verläuft. Dabei sind sowohl der distale als auch der proximale Kolben umlaufend gegenüber der Innenwandung des Spritzenkörpers abgedichtet, um ein Entweichen des Mediums aus dem zweiten Volumen, das sich zwischen den Kolben erstreckt, zu vermeiden.

Bei der diesbezüglichen Lösung kann zum Nachinjizieren des Mediums der proximale Kolben innerhalb des distalen Kolbens bewegt werden.

In dem von den Kolben begrenzten zweiten Volumen kann sich sterile Luft befinden, die zum Nachinjizieren bzw. Spülen benutzt wird.

Eine diesbezügliche Konstruktion bietet auch die Möglichkeit, dass ein Medium in den Zwischenraum zwischen den Kolben aufgezogen wird. Dies ist dann möglich, wenn der proximale Kolben in Richtung des distalen Kolbens verstellt wird, um anschließend das erste Betätigungselement mit dem proximalen Kolben zurückzuziehen, wobei das Volumen zwischen dem Kolben vergrößert und somit ein Medium aufgezogen werden kann. Hierzu weist der distale Kolben eine entsprechende Öffnung wie Schlitz auf, die die Funktion eines Ventils ausüben sollte.

Um beim Zurückziehen des Betätigungselementes und damit des proximalen Kolbens ein Herausziehen aus dem Spritzenkörper zu vermeiden, ist im Spritzenkörper ein Retentionsring vorgesehen, um den proximalen Kolben nach Erreichen einer Ausgangsposition zu verrasten. Beim Zurückziehen wird der distale Kolben mitbewegt, so dass gleichzeitig die Wirksubstanz aufgezogen werden kann.

Um beim axialen Verschieben der aus den Kolben bestehenden Einheit bis zum Anliegen des distalen Kolbens an dem Ansatz bzw. der Begrenzungswandung eine Relativbewegung zwischen den Kolben auszuschließen, ist vorgesehen, dass die Kolben zueinander verrastet sind, insbesondere durch Eingreifen von zumindest in einem der Kolben vorhandenem Vorsprung in eine angepasste Aufnahme in dem anderen Kolben. Alternativ kann ein Fixieren zueinander auch durch eine vorgegebene Haftreibung erzielt werden.

Eine weitere Ausgestaltung sieht vor, dass der distale Kolben über einen axial zusammendrückbaren, insbesondere balgartigen, Körper verbunden ist, wobei der Körper als Hohlkörper ausgebildet und umfangsseitig geschlossen ist.

Dabei besteht die Möglichkeit, dass der Körper peripher das zweite Volumen umschließt oder das zweite Volumen zwischen dem Hohlkörper und dem Spritzenkörper eingeschlossen ist.

Der aus elastischem Material bestehende Körper kann distalseitig in einen Abschnitt übergehen, der den distalen Kolben bildet. Proximal ist der Körper mit der Kolbenstange verbunden und gegenüber dem Spritzenkörper innenseitig abgedichtet. Dieser Bereich bildet dann die Funktion des ersten oder proximalen Kolbens, wenn sich das nachzuinjizierende Medium zwischen Außenseite des elastischen Körpers und Innenwandung des Spritzenkörpers befindet. Wird demgegenüber das nachzuinjizierende Medium von dem Körper umschlossen, so bildet der den von dem Körper umschlossenen Innenraum begrenzende Abschnitt der Kolbenstange den proximalen oder ersten Kolben, entlang dem sich ggf. auch der elastische Körper erstreckt.

Der die Kolben verbindende Bereich des aus elastischem Material bestehenden Körpers weist im nicht zusammengedrückten Zustand im Schnitt die Form von zwei kreisbogenförmigen in Bezug auf den Spritzenkörper konkav verlaufenden Abschnitten auf, wobei ggf. mittiger Abschnitt zumindest bereichsweise vorzugsweise parallel zur Längsachse des zylindrischen Spritzenkörpers verläuft. Folglich ist der Querschnitt des Körpers in Längsachsenrichtung betrachtet im Mittenbereich kleiner als in den Stirnrandbereichen.

Durch den insbesondere balgartigen Körper ist eine elastische Verbindung zwischen den Kolben gegeben. Die Spritze kann dabei entweder bereits mit zwischen den Kolben befindlichem nachzuinjizierenden Medium wie steriler Luft angeliefert werden oder vor Ort kann ein Befüllen erfolgen, sofern das Medium von dem Körper aufgenommen werden soll.

Um das zweite Volumen zu befüllen, ist es zunächst erforderlich, dass der die Kolben verbindende elastische, quasi federvorgespannte Körper entweder zusammengedrückt ist, um sodann durch Zurückziehen des proximalen Kolbens oder aufgrund selbststätigen Entspannens des Körpers der sich vergrößernden Raum zwischen den Kolben mit insbesondere steriler Luft befüllt wird. Durch weiteres Zurückziehen kann sodann die Wirksubstanz in den Raum zwischen dem Ansatz und der distal verlaufenden Stirnfläche des distalen Kolbens aufgezogen werden.

Bei den zuvor beschrieben Lösungen kann der distale Kolben derart ausgestaltet sein, dass ein Befüllen des Zwischenraums mit einem Medium, insbesondere steriler Luft, zwischen den Kolben erfolgt, also das Medium durch den distalen Kolben angesaugt werden kann. Unabhängig hiervon ist der distale Kolben derart ausgestaltet, dass bei Bewegung des proximalen Kolbens zu dem distalen Kolben in distaler Richtung das in dem Zwischenraum vorhandene Medium über den distalen Kolben abgegeben wird. Hierzu ist insbesondere vorgesehen, dass in dem Kolben eine Öffnung vorhanden ist, die z. B. durch ein hydrophobes Filter abgedeckt ist. Auch die Möglichkeit des Verschließens mittels einer Membran, die z. B. aufgrund des beim Bewegen des proximalen Kolbens in Richtung des distalen Kolbens zerstört wird, ist möglich. Eine Verbindung kann auch dadurch hergestellt werden, dass vom distalen Bereich des Spritzenkörpers sich ein Vorsprung in axialer Richtung erstreckt, der eine Öffnung ausbildet bzw. im Ausführungsbeispiel eine eine Öffnung verschließende Membran durchsticht.

Die Kolben sind durch einen elastischen Körper miteinander verbunden. Die Erfindung sieht vor, dass dann, wenn das Nachinjiziermedium injiziert ist, ein Zurückgleiten des proximalen Kolbens unterbunden wird. Dies kann durch z. B. Verrastung oder Klemmung oder Reibschluss erfolgen, so dass ein Zurückbewegen aufgrund der Vorspannung durch den elastischen Körper nicht möglich ist, wodurch andernfalls das Risiko besteht, dass Blut gezogen wird.

Es besteht die Möglichkeit, dass der proximale Kolben und/oder der elastische Körper fixiert wird, vorzugsweise durch Reibschluss.

Insbesondere ist vorgesehen, dass von dem proximalen Kolben ein in Längsachsenrichtung des Spritzenkörpers sich erstreckender Vorsprung ausgeht, der bei zusammengedrücktem elastischem Körper in dem Ansatz wie Luerkegel insbesondere durch Reibschluss fixierbar ist.

Es bezieht sich die Erfindung somit auch auf eine Spritze zur Injektion einer Wirksubstanz, wobei in einem Spritzenkörper zwei axial verschiebbare mechanisch verbundene Kolben vorgesehen sind, wobei distaler Kolben ein Hohlkörper oder ein Abschnitt von diesem ist und bei zusammengedrücktem Hohlkörper der proximale Kolben durch ein von diesem ausgehenden und sich in einem Ansatz des Spritzenkörpers erstreckenden Vorsprung fixiert ist, insbesondere durch Reibschluss. Somit kann nach erfolgter Injektion und fehlender Krafteinwirkung auf den proximalen Kolben dieser nicht zurückgleiten.

In Weiterbildung besteht die Möglichkeit, dass der vorzugsweise eine dorn- oder stiftförmige oder im Schnitt eine stern- oder kreuzförmige Geometrie aufweisende Vorsprung beim Zusammendrücken des elastischen Hohlkörpers diesen penetriert zur Herstellung einer Verbindung zwischen dem von dem Hohlkörper umgebenen zweiten Volumen und dem Ansatz.

Damit ein Strömen des Nachinjiziermediums durch den Ansatz aufgrund des sich in diesem erstreckenden Vorsprung nicht unterbunden wird, ist bevorzugterweise vorgesehen, dass der Vorsprung in seiner Außenseite zumindest eine vorzugsweise in Längsrichtung des Vorsprungs verlaufende Vertiefung wie Nut aufweist. Insbesondere ist vorgesehen, dass mehrere in Längsrichtung verlaufende Vertiefungen wie Nuten gleichmäßig verteilt um den Umfang des Vorsprungs vorhanden sind.

Ein weiterer Vorschlag sieht vor, dass ein Zurückgleiten des proximalen Kolbens durch Entspannen des elastischen Hohlkörpers nach Injizieren des Nachinjiziermediums dadurch unterbunden wird, dass ein Verrasten des Hohlkörpers und/oder des proximalen Kolbens erfolgt. Auch oder ergänzend besteht die Möglichkeit, dass ein Zurückgleiten aufgrund des in dem elastischen Körper umgebenen Raum herrschenden Unterdrucks unterbunden wird, da hierdurch bedingt ein Ausdehnen des elastischen Körpers nicht möglich ist.

Durch all diese Maßnahmen ist sichergestellt, dass nach dem Nachinjizieren ein Zurückgleiten des proximalen Kolbens unterbunden ist, so dass Blut aus dem Körper, in dem die Spritze gesetzt ist, nicht gezogen werden kann.

Die Erfindung zeichnet sich des Weiteren dadurch aus, dass der proximale Kolben den distalen Abschnitt einer das erste Betätigungselement bildenden Kolbenstange bildet, die vorzugsweise abschnittsweise an Innenseite des Zylinderkörpers anliegt. Insoweit werden konstruktive Lösungen vorgeschlagen, die von anderen Kolbenspritzen bekannt sind.

Die Erfindung zeichnet sich auch durch die Verwendung einer Spritze mit zumindest einigen der zuvor erläuterten Merkmalen oder einer auszuwählenden Kombination dieser zur Injektion einer Wirksubstanz sowie zum Nachinjizieren eines Mediums aus.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von bevorzugten Ausführungsbeispielen.

Es zeigen:
- Fig. 1: einen Schnitt durch eine erste Ausführungsform einer erfindungsgemäßen Spritze,
- Fig. 2 - 4: Ausschnitte der Spritze gemäß Fig. 1 in verschiedenen Arbeitsstellungen,
- Fig. 5: einen Ausschnitt der Spritze gemäß Fig. 1 - 4,
- Fig. 6 - 8: Ausschnitte einer zweiten Ausführungsform einer Spritze in verschiedenen Arbeitsstellungen,
- Fig. 9: einen Ausschnitt einer dritten Ausführungsform einer erfindungsgemäßen Spritze und
- Fig. 10 - 12: eine zu der zweiten Ausführungsform gemäß Fig. 6 - 8 alternative Ausführungsform einer Spritze in verschiedenen Arbeitsstellungen und
- Fig. 13 - 20: Varianten der Spritze gemäß der Fig. 6 - 8.

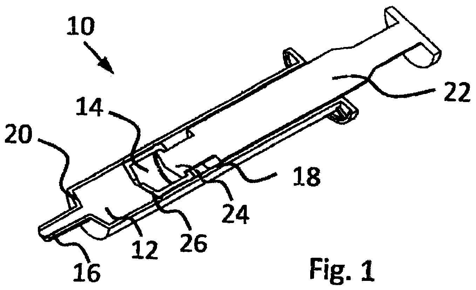

In Fig. 1 ist rein prinzipiell ein Längsschnitt durch eine Spritze 10 dargestellt, mit der zunächst eine Wirksubstanz 12 wie medizinisches Mittel und sodann ein Medium wie sterile Luft 14 nachinjiziert werden soll, um Restwirksubstanz, die sich zumindest in einem im distalen Bereich des Spritzenkörpers 18 verlaufenden Ansatz 16 und ggf. einer von dem Ansatz 16 ausgehenden Kanüle, einem Katheter oder sonstigem Schlauchsystem befindet, vollständig zu verabreichen. Der Ansatz 16 geht von einer distal verlaufenden Bodenwandung 20 des zylindrischen Spritzenkörpers 18 auf. Die Bodenwandung 20 wird auch als distale Begrenzungswandung bezeichnet.

Anstelle des Ansatzes 16 kann auch nur eine Öffnung vorhanden sein, über die über geeignete Verbindungsmittel das medizinische Mittel abgegeben wird.

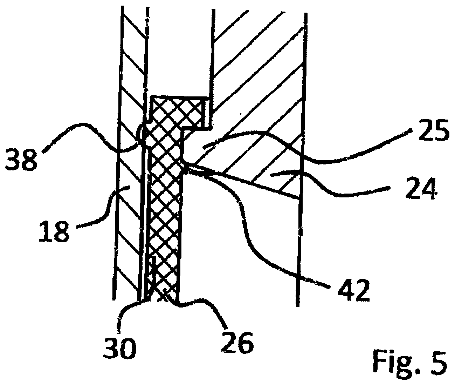

In axialer Richtung des Spritzenkörpers 18 ist eine Kolbenstange 22 verstellbar, die in den Ausführungsbeispielen distal als Kolben 24 ausgebildet ist, der auch als proximaler Kolben oder erster Kolben bezeichnet wird. Wird die Kolbenstange 22 im Ausführungsbeispiel an der Innenseite des Zylinderkörpers 18 gleitend geführt, so können auch andere Konstruktionen vorgesehen sein, die von Spritzen bekannt sind. Der proximale Kolben 24 ist mit einem distal verlaufenden oder zweiten Kolben 26 mechanisch gekoppelt, der als Hohlkolben ausgebildet ist und eine stirnseitige Bodenwandung 28 und eine als Hohlzylinder ausgebildete Umfangswandung 30 umfasst. Proximal, also im freien Stirnbereich, ist die Umfangswandung 30 radial nach innen, also in Richtung der Längsachse des Kolbens 26 und damit der Längsachse 32 des Spritzenkörpers 18 abgewinkelt. Der Randbereich ist mit dem Bezugszeichen 34 gekennzeichnet. Ist der Randbereich 34 vorzugsweise umlaufend abgewinkelt ausgebildet, kann der Rand auch nur abschnittsweise abgewinkelt sein. Der abgewinkelte Abschnitt bzw. Rand 34 erstreckt sich in eine entsprechend angepasste axiale Aussparung 36 in der Umfangswandung des ersten Kolbens 24, so dass der erste Kolben 24 zu dem zweiten Kolben 26 axial verstellbar ist. Der zweite Kolben 26 ist umfangsseitig gegenüber der Innenwandung des Spritzenkörpers 18 abgedichtet (siehe Bereich 38 in Fig. 5).

Aus der Detaildarstellung gemäß Fig. 5 wird auch erkennbar, dass der innerhalb des Hohlkolbens, also zweiten Kolbens 26 verlaufende Abschnitt 25 des ersten Kolbens 24 über z. B. einen umlaufenden Wulst 42 fixiert ist, um bei einem axialen Verstellen der Kolbenstange 22 in Richtung des Ansatzes 16 die Kolben 24, 26 als Einheit so lange zu verstellen, bis die Stirn- oder Bodenwandung 28 des zweiten Kolbens 26 auf der Begrenzungswandung 20 aufstößt. Bei weiterer axialer Krafteinleitung wird der durch den Wulst 42 gebildete Widerstand gegen ein axiales Verstellen der Kolbenstange 22 und damit des ersten Kolbens 24 in axialer Richtung überwunden, so dass der erste Kolben 24 innerhalb des zweiten Kolbens 26 axial verstellt werden kann, so dass das in dem Innenraum 43 zwischen den Kolben 24, 26 vorhandene Medium verdrängt wird und über eine in der Bodenwandung 28 des zweiten Kolbens 26 vorhandene Öffnung 44 austreten kann, um die in dem Ansatz 16 und in der mit diesem verbundenen Kanüle, Katheter, Schlauchsystem noch vorhandene Wirksubstanz in einen Körper vollständig zu injizieren.

Bei dem axialen Verstellen der Kolbenstange 22 mit den eine Einheit bildenden Kolben 24, 26 wird die in dem als erstes Volumen bezeichneten Raum 41 zwischen Außenseite der Bodenwandung 28 des zweiten Kolbens 26 und Innenseite der Begrenzungswandung 20 vorhandene Wirksubstanz über den Ansatz 16 abgegeben, um in einen Körper injiziert zu werden.

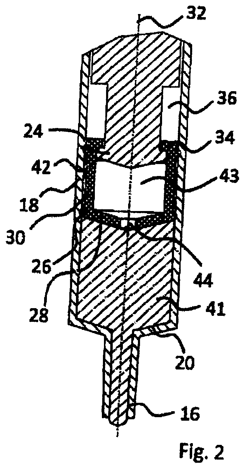

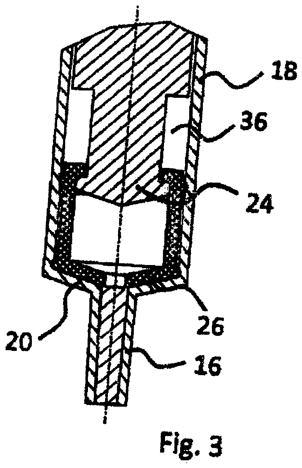

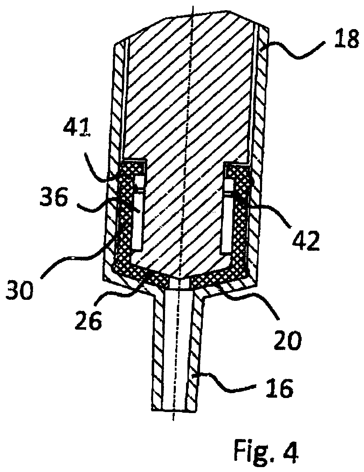

Die Verfahrensabfolge ergibt sich aus den Fig. 2, 3 und 4. Im Ausgangszustand befindet sich in dem zwischen der Außenfläche der Bodenwandung 28 des zweiten Kolbens 26 und dem Spritzenkörper 18 bis zum Ansatz 16 vorhandenen ein erstes Volumen 41 bildenden Bereich das zu injizierende Medikament (Fig. 2). Um dieses zu verabreichen, wird die Kolbenstange 22 axial in Richtung der Begrenzungswandung 20 verstellt, ohne dass eine Relativbewegung zwischen dem ersten und zweiten Kolben 24, 26 erfolgt. Sobald der zweite Kolben 26 mit seiner Bodenwandung 28 auf der Begrenzungswandung 20 aufliegt (Fig. 3), wird durch weitere axiale Krafteinleitung auf die Kolbenstange 22 der erste Kolben 24 durch Überwinden der Wulst 42 in Richtung des Ansatzes 16, also in Richtung der Innenseite der Begrenzungswandung 28 verstellt, so dass das in dem als zweites Volumen bezeichneten Zwischenraum 43 zuvor vorhandene Medium über die Öffnung 44 abgegeben wird (Fig. 4). Somit wird der in dem System aus Spritze 10 und Kanüle bzw. Katheter bzw. Schlauchsystem noch vorhandene Rest an Medikament freigespült. Dabei sollte das zweite Volumen 43 auf vorzugsweise 0,5 ml maximal begrenzt bzw. eingestellt sein, so dass beim Applizieren von Luft als Medium eine Luftembolie ausgeschlossen ist. Als Medium kommt aber auch z. B. eine Kochsalzlösung in Frage.

Durch das Freispülen ist gewährleistet, dass kein Totvolumen an Medikament in der Spritze 10 und der ggf. mit dieser verbunden Kanüle bzw. Katheter oder sonstigem Schlauchsystem verbleibt.

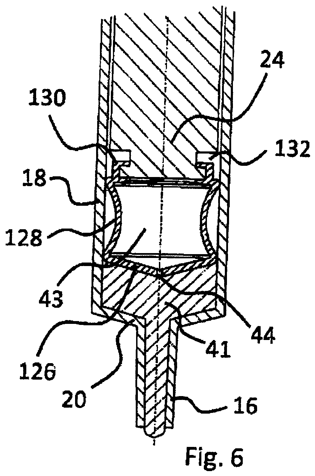

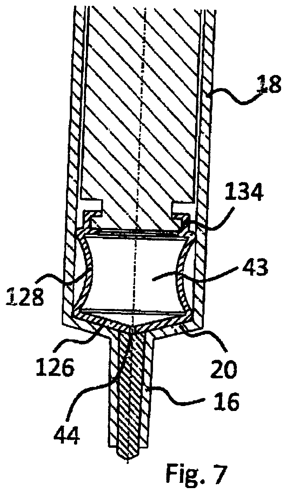

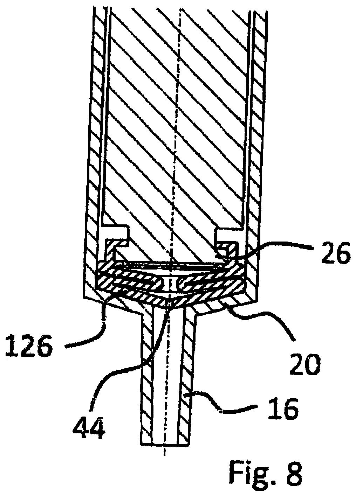

Den Fig. 6 - 8 ist eine weitere Ausführungsform der erfindungsgemäßen Lehre zu entnehmen. Dabei werden entsprechend den Fig. 1 - 5 für gleiche Elemente gleiche Bezugszeichen gewählt.

Abweichend von der Ausführungsform der Fig. 1 - 5 wird die Relativverschiebung eines distal verlaufenden, also zweiten Kolbens 126 zu dem ersten, also proximalen

Kolben 24 durch ein elastisches Element wie Gummibalg 128 erreicht, der die mechanische Kopplung zwischen dem ersten und zweiten Kolben 24, 126 sicherstellt. Der Balg 128 mit dem zweiten Kolben 126, der ein bodenseitiger Abschnitt des Balgs 128 sein kann, umschließt den das zweite Volumen 43 bildenden Raum, in dem ein Nachspülmedium wie sterile Luft vorhanden sein kann. Um den Faltenbalg 128 mit dem ersten Kolben 24 zu verbinden, weist der Balg 128 in seinem proximalen Bereich einen nach innen abgewinkelten Rand 130 auf, der in eine entsprechende Aussparung 132 im ersten Kolben 24 festlegbar und fixierbar ist. Der Balg 128 kann gegenüberliegend zu dem zweiten Kolben 126 offen oder verschlossen sein, wie dies der zeichnerischen Darstellung zu entnehmen ist (Abschnitt 134). Ungeachtet dessen ist der Faltenbalg 128 bzw. der zweite Kolben 126 umfangsseitig gegenüber der Innenwandung des Spritzenkörpers 18 abgedichtet. Zwischen dieser Abdichtung und der Begrenzungswandung 20 des Innenraums des Spritzenkörpers 18 ist in zuvor beschriebener Weise das zu injizierende Medikament vorhanden, das durch axiales Verstellen des ersten Kolbens 24 in Richtung der Begrenzungswand 20 abgegeben wird. Liegt der zweite Kolben 126 außenseitig an der Begrenzungswandung 20 an, wird bei weiterem axialen Verstellen des ersten Kolbens 24 der Faltenbalg 128 zusammengedrückt, um das von diesem umgebene Medium wie sterile Luft über die in dem zweiten Kolben 126 vorhandene Öffnung 44 abzugeben und somit das gewünschte Nachinjizieren zu ermöglichen.

Aus den Fig. 6 - 8 wird erkennbar, dass ein ringförmiger proximal verlaufender Bereich des Faltenbalgs 128 einen flanschartigen distal verlaufenden Abschnitt des ersten Kolbens 24 umschließt, um ein axiales Verschieben des Faltenbalgs 128 entlang Umfangswandung des ersten Kolbens 24 zu vermeiden.

Die Verfahrensschritte des Injizierens und Nachinjizierens ergeben sich selbsterklärend aus einem Vergleich der Fig. 6 - 8.

Um zu verhindern, dass nach Injizieren des Nachinjiziermediums, also nach Entleeren des zweiten Volumens 43, der proximale oder erste Kolben 24 aufgrund der in dem Gummibalg 128 gespeicherten Vorspannung zurückgedrückt wird, wodurch eine Sogwirkung mit der Folge entstehen würde, dass Blut über den Ansatz 16 angesaugt werden könnte, ist vorgesehen, dass der proximale Kolben 24 bzw. der diesen mit dem distalen Kolben 126 verbindende elastische Körper 128 nach Entleeren des zweiten Volumens 43 verrastet oder in anderer geeigneter Art fixiert wird, so dass ein Zurückgleiten des proximalen Kolbens 24 unterbunden ist. Gleichwirkend ist ein Verrasten des Faltenbalgs 128 in zusammengedrückter Stellung.

Auch besteht die Möglichkeit, dass ein Fixieren durch den Unterdruck erfolgt, der in dem zwischen dem ersten Kolben 24, dem Gummibalg 128 und dem distalen Kolben 126 begrenzten Innenraum durch das Herausdrücken des Nachinjiziermediums entsteht, wodurch ein Zurückdrücken des proximalen Kolbens 24 unterbunden wird.

Alternativ oder ergänzend besteht die Möglichkeit, dass die Öffnung, über die das Nachinjiziermedium abgegeben wird, über ein Rückschlagventil verschließbar ist, so dass ein Ansaugen und damit Entspannen des Faltenbalgs 128 nicht möglich ist.

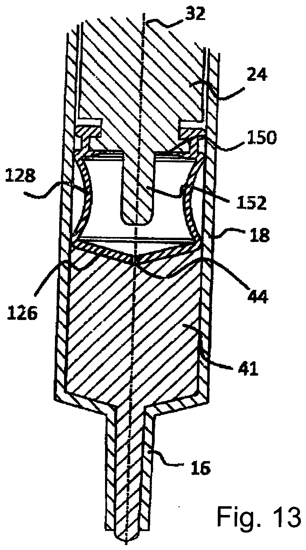

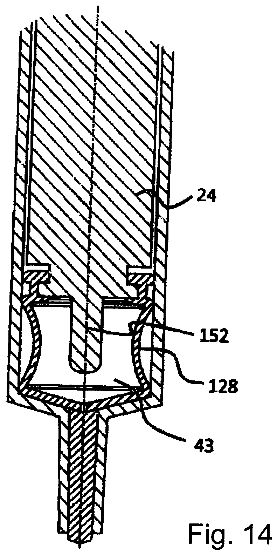

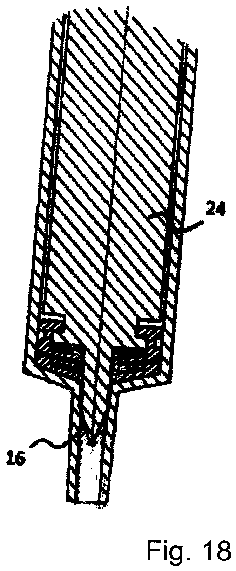

Besonders bevorzugte Lösungen, durch die ein Zurückgleiten des proximalen Kolbens 24 nach der Nachinjektion unterbunden wird, ergeben sich aus den Fig. 13 - 20. Diese stellen Varianten der Spritze gemäß der Fig. 6 - 8 dar, so dass für gleiche Elemente gleiche Bezugszeichen verwendet werden.

Die Varianten der Fig. 13 - 20 ermöglichen es, dass nach der Nachinjektion, also dem Entleeren des zweiten Volumens 43, das von dem den zweiten Kolben 126 bildenden elastischen Element wie Gummibalg 128 umgeben ist, ein Zurückgleiten des ersten oder proximalen Kolbens 24 aufgrund eines Ausdehnen des zusammengedrückten Gummibalgs 128 ausgeschlossen ist. Somit kann über den insbesondere als Luerkegel ausgebildeten Ansatz 16 und z. B. einen von diesem ausgehenden Schlauch kein Blut aus einer Vene gezogen werden. Dies wird durch die nachstehend erläuterten Maßnahmen ermöglicht.

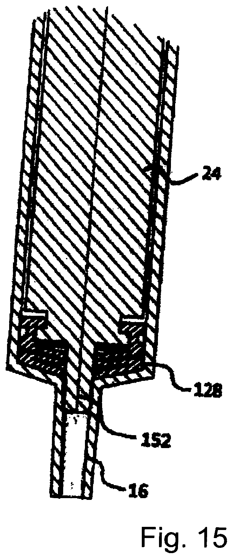

Nach dem Ausführungsbeispiel der Fig. 13 - 15 ist erfindungsgemäß vorgesehen, dass von dem proximalen Kolben 24, und zwar dessen senkrecht zur Längsachse 32 des Zylinderkörpers 18 verlaufenden Stirnfläche 150, ein sich in Richtung der Längsachse 32 erstreckender Vorsprung 152 ausgeht, der durchmessermäßig an den Innendurchmesser des Ansatzes 16 derart angepasst ist, dass beim Eindringen des Vorsprungs 152 in den Ansatz 16 bei zusammengedrücktem Balg 128 insbesondere ein Festklemmen durch Reibschluss erfolgt, wie durch die Fig. 15 verdeutlicht wird. Hierdurch wird der Vorsprung 152 und damit der proximale Kolben 24 fixiert, so dass sich der Faltenbalg 128 nicht ausdehnen kann, wodurch andernfalls ein Unterdruck in dem Ansatz 16 aufgebaut werden könnte mit der Folge, dass Blut gezogen wird.

Die Fig. 13 - 15 verdeutlichen wiederum die Abfolge bei der Nutzung der Spritze, d. h., zunächst das Injizieren des in dem ersten Volumen 41 vorhandenen zu injizierenden Medikaments und sodann anschließend das Injizieren des in dem zweiten Volumen 43 vorhandenen Nachinjiziermediums wie Luft. Hierzu weist die als zweiter Kolben 126 bezeichnete distal verlaufende stirnseitige Begrenzungswand des Balgs 128 eine entsprechende Öffnung 44 auf, über die das Nachinjiziermedium beim Zusammendrücken des Balgs 128 ausgestoßen werden kann.

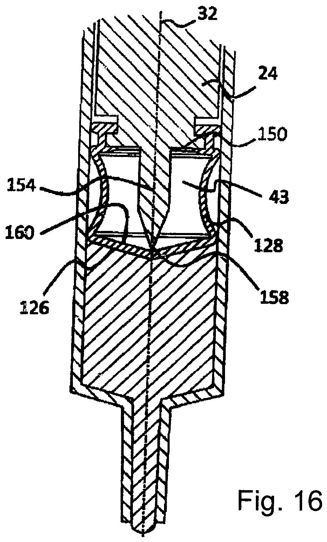

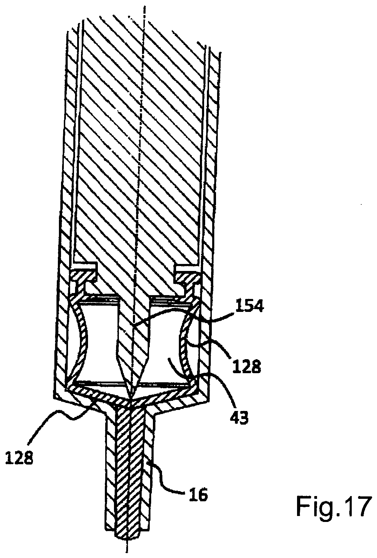

Die Ausführungsformen der Fig. 16 - 18 unterscheiden sich von denen der Fig. 13 - 15 dahingehend, dass die den zweiten Kolben 126 bildende distale Begrenzungswandung des Balgs 128 vollständig geschlossen ist, also das Nachinjiziermedium in dem zweiten Volumen 43 vollständig von dem Balg 128 und der stirnseitig verlaufenden Begrenzungsfläche 150 des ersten oder proximalen Kolbens 24 umgeben ist. Der in Längsachsenrichtung verlaufende Vorsprung 154, der von der Stirnfläche 150 ausgeht, unterscheidet sich in der Geometrie von dem Vorsprung 152 des Ausführungsbeispiels der Fig. 13 - 15 dahingehend, dass ersterer spitz zuläuft, also eine Penetrationsspitze 158 aufweist, mittels der die Wandung 126 durchstochen wird, wenn der Balg 128 entleert, also das in diesem vorhandene Nachinjiziermedium über den Ansatz 16 ausgestoßen werden soll. Daher weist der Vorsprung 156 eine Längenerstreckung auf, die in etwa dem Abstand entspricht zwischen der Begrenzungsfläche 150 und innerer Seite 160 der den zweiten Kolben 126 bildenden Begrenzungswandung des Balgs 128 bei nicht zusammengedrücktem Balg 128, so dass unmittelbar bei Beginn des Zusammendrückens des Balgs 128 (Fig. 17) die Spitze 158 die distale Wandung des Balgs 128, also den zweiten Kolben 126, durchsetzen kann. Somit wird das von dem Balg 128 umgebene zweite Volumen 43 mit dem Lumen des Ansatzes 16 verbunden und das Nachinjiziermedium kann ausströmen.

Der Querschnitt des Vorsprungs 154 ist entsprechend dem Ausführungsbeispiel der Fig. 13 - 15 gleichfalls an den Innenquerschnitt des Ansatzes 16 angepasst, so dass ein klemmendes Fixieren nach Entleeren des zweiten Volumens 43, also Zusammendrückens des Balgs 128 erfolgt, ohne dass anschließend ein Zurückgleiten möglich ist.

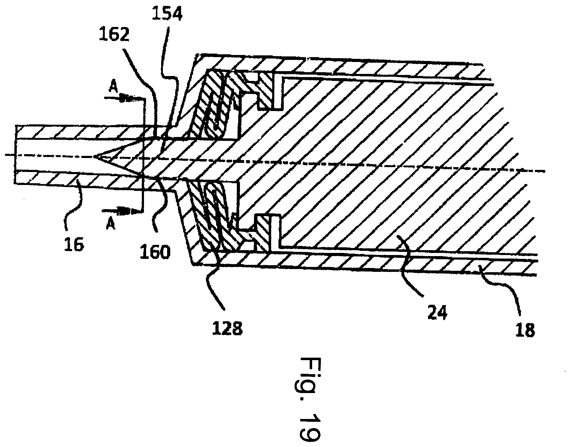

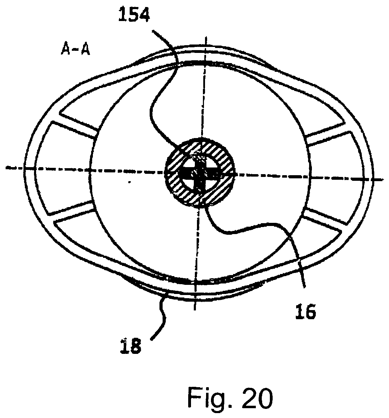

Um sicherzustellen, dass beim Eindringen des Vorsprungs 152 bzw. 154 in den Ansatz 16 das Nachinjiziermedium über den Ansatz 16 strömen kann, ist entsprechend der Darstellung der Fig. 19, 20 vorgesehen, dass der im Ausführungsbeispiel dargestellte Vorsprung 154 in Längsachsenrichtung verlaufende Vertiefungen aufweist, über die das Nachinjiziermedium in den Ansatz 16 hineinströmen kann.

Im Ausführungsbeispiel weist der Vorsprung 154 im Schnitt eine Kreuzgeometrie auf, wie die Schnittdarstellung A-A in Fig. 20 verdeutlicht. Ungeachtet dessen kann der Vorsprung 154 durch Reibschluss an der Innenwandung des Ansatzes 46 fixiert werden, um entsprechend der erfindungsgemäßen Lehre ein Zurückgleiten des proximalen Kolbens 24 durch Ausdehnen des Balgs 128 auszuschließen.

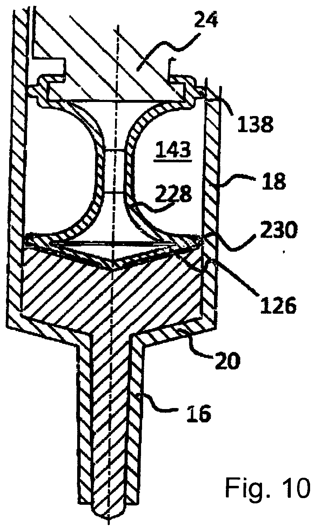

Eine zu der Spritze gemäß der Fig. 6 - 8 alternative Ausführungsform ist den Fig. 10-12 zu entnehmen, wobei für gleiche Elemente gleiche Bezugszeichen verwendet werden. Um Wiederholungen zu vermeiden, wird auf die Erläuterung zu den Fig. 6 - 8 verwiesen. Abweichend von dieser Ausführungsform umschließt bzw. umgibt ein elastisch zusammendrückbarer Balg 228, der zum einen von dem ersten Kolben 24 ausgeht und zum anderen distal den zweiten oder distalen Kolben 126 aufweist oder diesen bildet, nicht das zweite Volumen. Dieses verläuft vielmehr zwischen Außenseite des Balgs 228 und Innenseite des Spritzenkörpers 18. Dieser Bereich ist mit dem Bezugszeichen 143 gekennzeichnet. Damit das in dem zweiten Volumen 143 vorhandene nachzuinjizierende Medium wie sterile Luft beim Verstellen des ersten Kolbens 24 in Richtung der Begrenzungswandung 20 nicht entweichen kann, ist der Balg 228 in seinem proximalen Bereich gegenüber dem Spritzenkörper 18 abgedichtet. Dies wird durch eine umlaufende Wulst 138 symbolisiert.

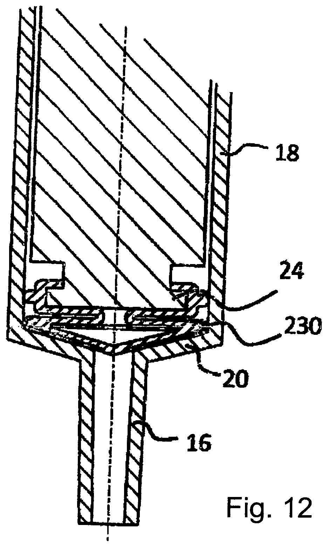

Damit aus dem zweiten Volumen 143 das vorhandene nachzuinjizierende Medium beim Zusammendrücken des Balgs 228 über den Ansatz 16 strömen kann (Fig. 12), weist der Balg 228 in seinem distalen Bereich, der an der Innenseite des Spritzenkörpers anliegt, Schlitze auf, die in radial verlaufende Vertiefungen 230 in der Außenfläche des zweiten Kolbens 126 übergehen.

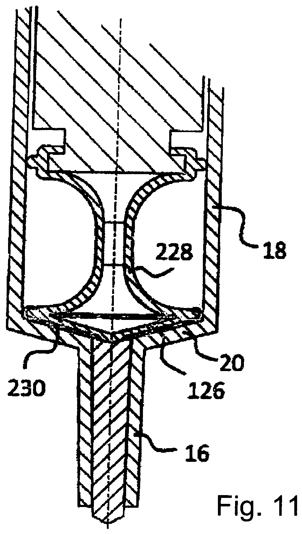

Wie sich aus den Schnittdarstellungen der Fig. 10 und 11 ergibt, weist der Balg 228 - wie entsprechend in etwa der Balg 128 - im Schnitt die Form von zwei kreisbogenartigen Abschnitten auf, die in Bezug auf den Zylinderkörper 18 konkav gestaltet sind und endseitig in die Kolben 24, 126 übergehen. Im Mittenbereich kann der Balg 228 parallel zur Längsachse des Spritzenzylinders 18 verlaufen, wie sich aus den Fig. 10 und 11 ergibt.

Damit beim Zusammendrücken des Balgs 228 (Fig. 11 nach Fig. 12) innerhalb des Balgs 228 vorhandene Luft entweichen kann, können entsprechende Öffnungen vorgesehen sein, die die Kolbenstange bzw. den Kolben 24 durchsetzen, ohne dass diese dargestellt sind.

Im Übrigen sind die Darstellungen gemäß Fig. 10 - 12 selbsterklärend, soweit die Funktion des Balgs 228 und dessen Geometrie betroffen sind.

Ein Fixieren des proximalen Kolbens 24 nach Herausdrücken des Nachinjiziermediums aus dem zweiten Volumen 143^{∗} kann entsprechend zuvor erfolgter Erläuterung gleichfalls erfolgen.

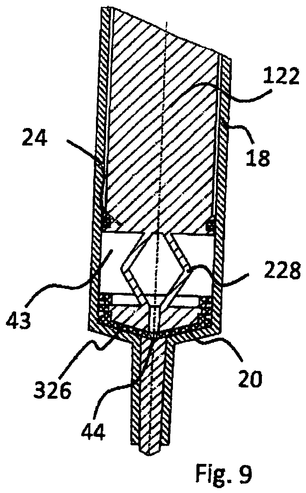

Eine weitere Ausführungsform einer erfindungsgemäßen Spritze ist der Fig. 9 zu entnehmen. Dabei ist der erste oder proximale Kolben 24, der abweichend von der Ausführungsform der Fig. 1 von einer die Innenwandung des Spritzenkörpers 18 nicht berührenden Kolbenstange 122 ausgeht, über Stegelemente 228 mit dem distalen Kolben 326 verbunden, die dann zerstörbar wie durchbrechbar sind, wenn der über die Verbindungselemente 228 mit dem ersten Kolben 24 verbundene zweite Kolben 326 auf der Begrenzungsfläche 20 des Spritzenkörpers 18 aufliegt und auf die Kolbenstange 122 weiterhin in Richtung der Begrenzungsfläche 20 eine Krafteinleitung erfolgt. Sodann kann der zwischen dem ersten Kolben 24 und dem zweiten Kolben 326 vorhandene Zwischenraum (zweites Volumen 43) über die in dem zweiten Kolben 326 vorzugsweise zentral verlaufende Öffnung 44 in das mit der Spritze verbundene System abgegeben werden.

Die Öffnung 44 kann als Schlitzventil ausgebildet sein. Gleiches gilt für die Ausführungsformen der zuvor erläuterten Ausführungsbeispiele. Es besteht auch die Möglichkeit, die Öffnung mit einem hydrophoben Filter zu versehen. Die Möglichkeit, die Öffnung mit einer Membran abzudecken, die zerstört wird - sei es durch Druckaufbau im zweiten Volumen 43 oder durch von der Begrenzungswandung 20 ausgehenden Vorsprüngen - ist eine weitere Möglichkeit, um im erforderlichen Umfang das im zweiten Volumen 43 vorhandene Medium wie insbesondere sterile Luft nachinjizieren zu können.

Ein Aufziehen des zum Nachinjizieren benötigten Mediums bzw. der Wirksubstanz selbst kann mit den Ausführungsformen der Fig. 1 - 8 und 10 - 12 gleichfalls erfolgen. Zum Aufziehen ist es zunächst erforderlich, dass der erste Kolben 24 in Richtung des zweiten Kolbens 26, 126 verstellt ist, so dass das von diesem begrenzte zweite Volumen 43 verkleinert ist. Sodann muss die Kolbenstange 22 zurückgezogen werden, um den Zwischenraum zwischen den Kolben 24, 26 bzw. 124, 126 zu vergrößern, wodurch ein Aufziehen des Mediums möglich wird. Es erfolgt quasi eine Verfahrensabfolge in der Reihenfolge Fig. 4, Fig. 3 bzw. Fig. 8, Fig. 7 bzw. Fig. 11, Fig. 10. Zum Auffüllen des ersten Volumens 41 liegt der zweite Kolben 26, 126 an der Begrenzungswand 20 an. Durch weiteres axiales Verstellen der Kolbenstange 22 von der Begrenzungswandung 20 weg kann sodann die Wirksubstanz aufgezogen werden.

## Patentansprüche

1. Spritze (10) zur Injektion einer Wirksubstanz, umfassend einen zylindrischen Spritzenkörper (18) mit im distalen Bereich vorhandener Begrenzungswandung (20), die in einen Ansatz (16), wie Luerkegel, übergeht bzw. eine Öffnung aufweist, über den bzw. die die Wirksubstanz abgebbar ist, zwei in dem Spritzenkörper axial verschiebbare mechanisch über einen elastischen Körper (128) verbundene Kolben (24, 26, 126) und ein Betätigungselement (22, 122) zum gemeinsamen Verschieben der Kolben, wobei vor der Injektion der Wirksubstanz distaler Kolben (26, 126) und die Begrenzungswandung (20) zueinander beabstandet sind und hierdurch gebildetes erstes Volumen (41) mit der Wirksubstanz gefüllt ist, wobei die Kolben bei Kraftbeaufschlagung des Betätigungselementes in Richtung der Begrenzungswandung als mechanisch gekoppelte Einheit bis zum Anliegen des distalen Kolbens an der Begrenzungswandung oder einem Anschlag verstellbar sind, wobei bei mechanisch gekoppelter Einheit der Kolben (24, 26, 126) einander zugewandte Innenflächen der Kolben zueinander beabstandet sind und hierdurch gebildetes zweites Volumen (43) mit einem Nachinjiziermedium gefüllt ist und nach Anliegen des distalen Kolbens (26, 126) an der Begrenzungswandung (20) bei weiterer axialer Krafteinleitung auf das Betätigungselement (22, 122,) die mechanische Verbindung zwischen den Kolben selbsttätig derart veränderbar ist, dass proximaler Kolben in Richtung des distalen Kolbens verstellbar ist, und wobei beim Verstellen des proximalen Kolbens in Richtung des distalen Kolbens das Nachinjiziermedium den distalen Kolben durchsetzt und/oder an diesem vorbeiströmt,
**dadurch gekennzeichnet,**
**dass** das zweite Volumen (43) mit einem Gas als das Nachinjiziermedium gefüllt ist und dass nach dem Injizieren des Nachinjiziermediums ein Zurückgleiten des proximalen Kolbens (24) durch Klemmung oder Reibschluss oder durch im von den Kolben (24, 126) und dem diese verbindenden elastischen Körper (128) umgebenen Raum herrschenden Unterdruck unterbunden ist, wobei dann, wenn das Zurückgleiten durch den Unterdruck unterbunden ist, der distale Kolben (126) in seiner distal verlaufenden Begrenzung zumindest eine Öffnung aufweist, die über ein Rückschlagventil verschlossen ist.

2. Spritze nach Anspruch 1,
**dadurch gekennzeichnet,**
**dass** der distale Kolben (26) ein Hohlkolben mit distal verlaufender Bodenwandung (28) und von dieser ausgehender, einen Hohlzylinder bildender Umfangswandung (30) mit stirnseitig zumindest abschnittsweise radial in Richtung der Längsachse (32) des Kolbens sich erstreckendem Randbereich (34), insbesondere umlaufendem nach innen abgewinkelten Rand, ist, der in eine den Randbereich aufnehmende in der Außenseite des proximalen Kolbens (24) axial verlaufende Aussparung (36) eingreift, die beabstandet zu zumindest distalem Rand des proximalen Kolbens verläuft.

3. Spritze nach Anspruch 1 oder 2,
**dadurch gekennzeichnet,**
**dass** beim Verschieben der Kolben (24, 26) als Einheit diese durch Wechselwirken eines zumindest in einem der Kolben vorhandenen Vorsprungs (42) mit einer angepassten Aufnahme in dem anderen Kolben oder durch Haftreibung zueinander beabstandet bleiben.

4. Spritze nach Anspruch 1,
**dadurch gekennzeichnet,**
**dass** die Kolben (24) durch axial verlaufende Abstandselemente (228) mechanisch gekoppelt sind, die nach Anliegen des distalen Kolbens auf der Begrenzungswandung (20) bzw. dem Anschlag bei weiterer Krafteinleitung zerstört werden.

5. Spritze nach Anspruch 1,
**dadurch gekennzeichnet,**
**dass** der distale Kolben (126) über einen axial zusammendrückbaren, vorzugsweise balgartigen Hohlkörper (128, 228) mit dem proximalen Kolben (24) verbunden ist.

6. Spritze nach zumindest einem der vorhergehenden Ansprüche,
**dadurch gekennzeichnet,**
**dass** der proximale Kolben (24) distaler Abschnitt einer das erste Betätigungselement bildenden Kolbenstange (22) ist, die vorzugsweise zumindest abschnittsweise an Innenseite des Spritzenkörpers (18) anliegt.

7. Spritze nach zumindest einem der vorhergehenden Ansprüche,
**dadurch gekennzeichnet,**
**dass** der distale Kolben (26, 126) in seiner distal verlaufenden Begrenzung zumindest eine Öffnung aufweist, die von einem hydrophoben Filterelement (446) oder einem zerstörbaren Membranelement verschlossen ist.

8. Spritze nach zumindest einem der vorhergehenden Ansprüche,
**dadurch gekennzeichnet,**
**dass** das Betätigungselement (22) mit dem proximalen Kolben (24) verbunden ist, in den die Verstellkräfte sowohl zum Verstellen der aus den Kolben (24, 26, 126) bestehenden Einheit als auch zum Verstellen des proximalen Kolbens zu dem distalen Kolben eingeleitet werden.

9. Spritze nach Anspruch 1,
**dadurch gekennzeichnet,**
**dass** das Nachinjiziermedium sterile Luft ist.

10. Spritze nach zumindest Anspruch 5,
**dadurch gekennzeichnet,**
**dass** distaler Bereich des zusammendrückbaren Hohlkörpers (228) der distale Kolben (126) ist.

11. Spritze nach zumindest einem der vorhergehenden Ansprüche,
**dadurch gekennzeichnet,**
**dass** bei zusammengedrücktem Hohlkörper (128, 228) der Hohlkörper in zusammengedrückter Stellung und/oder der proximale Kolben (24) fixierbar ist.

12. Spritze nach zumindest einem der vorhergehenden Ansprüche,
**dadurch gekennzeichnet,**
**dass** von dem proximalen Kolben (24) ein in Längsachsenrichtung des Spritzenkörpers (18) sich erstreckender Vorsprung (152, 154) ausgeht, der bei zusammengedrücktem Hohlkörper (128, 228) in dem Ansatz (16) wie Luerkegel insbesondere durch Reibschluss fixierbar ist.

13. Spritze nach Anspruch 12,
**dadurch gekennzeichnet,**
**dass** der Vorsprung (152, 154) insbesondere einer dorn- oder stiftförmigen oder im Querschnitt einer kreuz- oder sternförmigen Geometrie beim Zusammendrücken des Hohlkörpers (128, 228) diesen penetriert zur Herstellung einer Verbindung zwischen dem von dem Hohlkörper umgebenen zweiten Volumen (43) und dem Ansatz (16), wobei vorzugsweise der Vorsprung (154) in seiner Außenseite zumindest eine vorzugsweise in Längsrichtung des Vorsprungs verlaufende Vertiefung wie Nut aufweist.

## Claims

1. A syringe (10) for injecting an active substance, comprising a cylindrical syringe body (18) having a boundary wall (20) in the distal region, said boundary wall (20) merging into an extension (16), such as a luer cone, or comprising an opening via which extension or opening the active substance is dispensable, two plungers (24, 26, 126) which are axially movable in the syringe body and mechanically connected via an elastic body (128), and an actuator (22, 122) for commonly moving the plungers; wherein, prior to the injection of the active substance, the distal plunger (26, 126) and the boundary wall (20) are spaced apart from each other and a first volume (41) formed thereby is filled with the active substance, wherein, when force is applied to the actuator in the direction of the boundary wall, the plungers are displaceable as a mechanically coupled unit until the distal plunger contacts the boundary wall or a stop, wherein, when the plungers (24, 26, 126) are mechanically coupled as a unit, the inner surfaces of the plungers facing each other are spaced apart from each other and a second volume (43) formed thereby is filled with a post-injection medium and, when further axial force is applied to the actuator (22, 122) once the distal plunger (26, 126) contacts the boundary wall (20), the mechanical connection between the plungers is automatically changeable such that the proximal plunger is displaceable in the direction of the distal plunger, and wherein, when the proximal plunger is displaced in the direction of the distal plunger, the post-injection medium traverses the distal plunger and/or flows past the latter, **characterized in that**
the second volume (43) is filled with a gas as the post-injection medium and **in that**, once the post-injection medium is injected, the proximal plunger (24) is prevented from sliding back by clamping or frictional locking or by underpressure prevailing in the space surrounded by the plungers (24, 126) and the elastic body (128) connecting the plungers (24, 126), wherein, when sliding back is prevented by the underpressure, the distal plunger (126) comprises at least one opening in its distally extending boundary, said at least one opening being closed by a check valve.

2. The syringe according to claim 1,
**characterized in that**
the distal plunger (26) is a hollow plunger comprising a distally extending bottom wall (28) and a peripheral wall (30) that extends therefrom, forms a hollow cylinder and comprises an edge region (34), in particular a circumferential inwardly angled edge, extending on the face side at least in sections radially in the direction of the longitudinal axis (32) of the plunger, said hollow plunger engaging with a recess (36) that accommodates the edge region, axially extends in the outer side of the proximal plunger (24) and extends at a distance from at least the distal edge of the proximal plunger.

3. The syringe according to claim 1 or 2,
**characterized in that**
when being displaced as a unit, the plungers (24, 26) remain spaced apart from each other by means of the interaction of a projection (42) present in at least one of the plungers with a mating accommodation in the other plunger or by static friction.

4. The syringe according to claim 1,
**characterized in that**
the plungers (24) are mechanically coupled by axially extending spacers (228) which are destroyed when further force is applied once the distal plunger contacts the boundary wall (20) or the stop.

5. The syringe according to claim 1,
**characterized in that**
the distal plunger (126) is connected to the proximal plunger (24) via an axially compressible, preferably bellows-like, hollow body (128, 228).

6. The syringe according to at least one of the preceding claims,
**characterized in that**
the proximal plunger (24) is the distal portion of a plunger rod (22) forming the first actuator and contacting preferably at least in sections the inner side of the syringe body (18).

7. The syringe according to at least one of the preceding claims,
**characterized in that**
the distal plunger (26, 126) comprises at least one opening in its distally extending boundary, said opening being closed by a hydrophobic filter element (446) or a destructible membrane element.

8. The syringe according to at least one of the preceding claims,
**characterized in that**
the actuator (22) is connected to the proximal plunger (24), onto which the displacing forces both for displacing the unit consisting of the plungers (24, 26, 126) and for displacing the proximal plunger towards the distal plunger are applied.

9. The syringe according to claim 1,
**characterized in that**
the post-injection medium is sterile air.

10. The syringe according to at least claim 5,
**characterized in that**
the distal region of the compressible hollow body (228) is the distal plunger (126).

11. The syringe according to at least one of the preceding claims,
**characterized in that** ,
when the hollow body (128, 228) is compressed, the hollow body in its compressed position and/or the proximal plunger (24) is fixable.

12. The syringe according to at least one of the preceding claims,
**characterized in that**
a projection (152, 154) extending in the direction of the longitudinal axis of the syringe body (18) protrudes from the proximal plunger (24), which projection (152, 154), when the hollow body (128, 228) is compressed, can be fixed in the extension (16), such as a luer cone, in particular by frictional locking.

13. The syringe according to claim 12,
**characterized in that**
the projection (152, 154), in particular having a mandrel-shaped or pin-shaped or, in cross-section, a cruciform or star-shaped geometry, penetrates the hollow body (128, 228) when the latter is compressed in order to create a connection between the second volume (43) surrounded by the hollow body and the extension (16), wherein preferably the projection (154) comprises in its outer side at least one depression, such as a groove, preferably extending in the longitudinal direction of the projection.

## Revendications

1. Seringue (10) pour l'injection d'une substance active, comprenant un corps de seringue (18) cylindrique avec une paroi de délimitation (20) présentée dans la région distale se terminant par un embout (16), tel qu'un embout Luer Lock, ou présentant une ouverture, par lequel ou par laquelle la substance active est administrable, deux pistons (24, 26, 126) déplaçables axialement, raccordés mécaniquement par un corps élastique (128) dans le corps de seringue, et un élément d'actionnement (22, 122) pour le déplacement conjoint des pistons, où, avant injection de la substance active, le piston distal (26, 126) et la paroi de délimitation (20) sont espacés l'un de l'autre et un premier volume (41) ainsi formé est rempli de substance active, les pistons étant déplaçables dans la direction de la paroi de délimitation comme unité couplée mécaniquement par application de force de l'élément d'actionnement, jusqu'à contact du piston distal contre la paroi de délimitation ou contre une butée, les surfaces intérieures opposées l'une à l'autre des pistons étant espacées l'une de l'autre en cas d'unité de pistons (24, 26, 126) couplée mécaniquement et un deuxième volume (43) ainsi formé étant rempli d'un fluide de post-injection, et où, après contact du piston distal (26, 126) contre la paroi de délimitation (20), une application de force axiale étant poursuivie sur l'élément d'actionnement (22, 122), la liaison mécanique entre les pistons est modifiable automatiquement de telle manière que le piston proximal est déplaçable dans la direction du piston distal, et où, lors du déplacement du piston proximal dans la direction du piston distal le fluide de post-injection traverse le piston distal et/ou s'écoule sur celui-ci,
**caractérisé en ce que** le deuxième volume (43) est rempli d'un gaz en tant que fluide de post-injection et qu'après injection du fluide de post-injection, un coulissement de retour du piston proximal (24) est empêché par blocage ou frottement ou par la dépression régnant dans l'espace entouré par les pistons (24, 126) et le corps élastique (128) raccordant ceux-ci, le piston distal (126) présentant alors, lorsque le coulissement de retour est empêché par la dépression, au moins une ouverture obturée par une valve anti-retour dans sa délimitation à extension distale.

2. Seringue selon la revendication 1,
**caractérisée en ce que** le piston distal (26) est un piston creux à paroi inférieure (28) à extension distale et paroi périphérique (30) partant de celle-ci, formant un cylindre creux avec une zone de bordure (34), en particulier un bord périphérique plié vers l'intérieur s'étendant frontalement au moins en partie radialement dans la direction de l'axe longitudinal (32) du piston, lequel s'engage dans un évidement (36) à extension axiale recevant la zone de bordure à l'extérieur du piston proximal (24), ledit évidement s'étendant à intervalle au moins du bord distal du piston proximal.

3. Seringue selon la revendication 1 ou la revendication 2,
**caractérisée en ce qu'**en cas de déplacement des pistons (24, 26) en tant qu'unité, ceux-ci restent espacés l'un de l'autre par interaction d'une saillie (42) présentée au moins dans un des pistons avec une réception adaptée dans l'autre piston, ou par adhérence.

4. Seringue selon la revendication 1,
**caractérisée en ce que** les pistons (24) sont mécaniquement couplés par des éléments d'espacement (228) à extension axiale, lesquels sont détruits après contact du piston distal sur la paroi de délimitation (20) ou butée lorsqu'une force continue à être appliquée.

5. Seringue selon la revendication 1,
**caractérisée en ce que** le piston distal (126) est raccordé au piston proximal (24) par un corps creux (128, 228) axialement compressible, préférentiellement en forme de soufflet.

6. Seringue selon au moins une des revendications précédentes,
**caractérisée en ce que** le piston proximal (24) est un segment distal d'une tige de piston (22) formant le premier élément d'actionnement, laquelle repose préférentiellement au moins en partie contre la face intérieure du corps de seringue (18).

7. Seringue selon au moins une des revendications précédentes,
**caractérisée en ce que** le piston distal (26, 126) présente au moins une ouverture dans sa délimitation distale, laquelle est obturée par un élément de filtre (446) hydrophobe ou un élément de membrane destructible.

8. Seringue selon au moins une des revendications précédentes,
**caractérisée en ce que** l'élément d'actionnement (22) est raccordé au piston proximal (24) dans lequel sont appliquées les forces de déplacement pour le déplacement de l'unité formée par les pistons (24, 26, 126) ainsi que pour le déplacement du piston proximal vers le piston distal.

9. Seringue selon la revendication 1,
**caractérisée en ce que** le fluide de post-injection est de l'air stérile.

10. Seringue selon au moins la revendication 5,
**caractérisée en ce que** la région distale du corps creux compressible (228) est le piston distal (126).

11. Seringue selon au moins une des revendications précédentes,
**caractérisée en ce qu'**en cas de corps creux (128, 228) comprimé, le corps creux en position comprimée et/ou le piston proximal (24) est fixable.

12. Seringue selon au moins une des revendications précédentes,
**caractérisée en ce qu'**une saillie (152, 154) s'étendant dans le sens de l'axe longitudinal des corps de seringue (18) part du piston proximal (24), laquelle est en cas de corps creux (128, 228) comprimé fixable dans l'embout (16) tel qu'un embout Luer Lock, en particulier par frottement.

13. Seringue selon la revendication 12,
**caractérisée en ce que** la saillie (152, 154) ayant en particulier une géométrie en mandrin ou en goupille, ou en croix ou en étoile en section transversale, pénètre le corps creux (128, 228) lors de la compression de celui-ci pour réaliser une liaison entre le deuxième volume (43) entouré par le corps creux et l'embout (16), ladite saillie (154) présentant préférentiellement sur sa face extérieure au moins un évidement tel qu'une rainure s'étendant préférentiellement dans le sens de la longueur de la saillie.
